Europäisches Patentamt

European Patent Office

Office européen des brevets

(11) Numéro de publication: **0 362 004**
**A1**

## (12) DEMANDE DE BREVET EUROPEEN

(21) Numéro de dépôt: **89402402.5**

(22) Date de dépôt: **04.09.89**

(51) Int. Cl.5: **G02C 7/02 , A61F 2/16**

Le titre de l'invention a été modifié (Directives relatives à l'examen pratiqué à l'OEB, A-III, 7.3)

(30) Priorité: **06.09.88 FR 8811623**

(43) Date de publication de la demande:
**04.04.90 Bulletin 90/14**

(84) Etats contractants désignés:
**BE CH DE ES GB IT LI NL SE**

(71) Demandeur: **ESSILOR INTERNATIONAL, Cie Générale d'Optique**
**1 Rue Thomas Edison, Echat 902**
**F-94028 Creteil Cédex(FR)**

(72) Inventeur: **Mercier, Jean-Louis**
**3 Rue du Bon Puits**
**F-91640 Fontenay les Briis(FR)**

(74) Mandataire: **CABINET BONNET-THIRION**
**95 Boulevard Beaumarchais**
**F-75003 Paris(FR)**

(54) **Système optique combinant une lentille ophtalmique et une lentille intra-oculaire.**

(57) Il s'agit d'un système optique du genre comportant, en combinaison, deux lentilles, à savoir, d'une part une lentille ophtalmique (10) de forte puissance positive disposée devant l'oeil (12) à traiter, et, d'autre part, une lentille intra-oculaire (11) de forte puissance négative implantée dans cet oeil (12) après ablation de son cristallin.

Suivant l'invention, l'une au moins des surfaces de l'une et de l'autre de ces lentilles (10, 11), est une surface de révolution asphérique, et, de préférence, une surface de révolution asphérique ayant pour génératrice une conique.

Application à l'amélioration de la vision d'une personne atteinte de dégénérescence maculaire.

FIG.1

EP 0 362 004 A1

# Système optique, à lentille ophtalmique et lentille intra-oculaire, pour l'amélioration de la vision d'une personne atteinte de dégénérescence maculaire

La présente invention, qui fait suite à des travaux de Messieurs Georges BAIKOFF, Bernard GARNIER, et Yves MAIGRET, concerne d'une manière générale l'amélioration de la vision d'une personne atteinte de dégénérescence maculaire, c'est-à-dire d'une dégénérescence de la macula de la rétine de l'un et/ou l'autre de ses yeux.

Ainsi qu'on le sait, cette dégénérescence maculaire, qui n'affecte en général que les personnes agées de plus de 65 ans, se traduit par une baisse d'activité pour les bâtonnets de la zone concernée de la rétine, et il en résulte, pour les personnes qui en sont atteintes, l'impossibilité de lire en vision de près, et, en vision de loin, la perte d'une vision "propre", c'est-à-dire d'une vision exploitable pour une déambulation convenable.

En bref, il en résulte une certaine forme de cécité.

Pour améliorer la vision de personnes ainsi atteintes de dégénérescence maculaire, il a d'abord été proposé de disposer, devant l'oeil à traiter, aux lieu et place d'une simple lentille ophtalmique, un système optique comportant plusieurs de telles lentilles, du type par exemple d'une lunette de GALILEE, et propre à agrandir les images formées sur la rétine de cet oeil et à ainsi éclairer un nombre maximal de bâtonnets de celle-ci.

Mais, avec un tel système optique, l'agrandissement d'image envisageable est relativement modéré et, corollairement, et surtout, si une certaine vision est ainsi restaurée, cette restauration n'intervient que dans un champ limité, ce qui en minimise l'intérêt.

Toutes les lentilles constitutives de ce système optique restent cependant extérieures à l'oeil.

En outre, les systèmes optiques mettant ainsi en oeuvre une lunette du type lunette de GALILEE sont lourds et encombrants.

Il est maintenant également proposé un système optique faisant appel à l'oeil lui-même.

Sous des formulations diverses, ce système optique comporte, d'une manière générale, en combinaison, deux lentilles, à savoir, d'une part, une lentille ophtalmique de forte puissance positive disposée devant l'oeil à traiter, et, d'autre part, une lentille intra-oculaire de forte puissance négative implantée dans cet oeil en substitution à son cristallin.

Par exemple, cette lentille intra-oculaire peut être implantée dans la chambre postérieure de l'oeil.

En variante, elle peut être implantée dans la chambre antérieure de celui-ci.

Quoi qu'il en soit, l'agrandissement d'image obtenu avec un tel système optique peut être largement supérieur à celui obtenu avec un système optique extérieur à l'oeil, et le champ correspondant peut être lui aussi avantageusement plus important.

Mais, même s'il a été envisagé de doter d'une surface de révolution asphérique l'une des lentilles constitutives de ce système optique, en l'espèce la lentille ophtalmique disposée en avant de l'oeil, il subsiste, le plus souvent, avec un tel système optique, notamment lorsque, pour des raisons de facilité opératoires aussi bien que pour permettre ultérieurement une nouvelle intervention, la lentille intra-oculaire est implantée dans la chambre antérieure de l'oeil, des aberrations non négligeables, dont il résulte, inévitablement, une certaine dégradation de la qualité des images obtenues.

La présente invention est fondée sur l'observation qu'il est au contraire possible d'améliorer très sensiblement la qualité de ces images en dotant d'une surface de révolution asphérique l'une et l'autre des lentilles constitutives d'un tel système optique.

Elle a ainsi pour objet un système optique pour l'amélioration de la vision d'une personne atteinte de dégénérescence maculaire, du genre comportant, en combinaison, deux lentilles à savoir, d'une part, une lentille ophtalmique de forte puissance positive disposée devant l'oeil à traiter, et, d'autre part, une lentille intra-oculaire de forte puissance négative implantée dans cet oeil après ablation de son cristallin, ce système optique étant d'une manière générale caractérisé en ce que l'une au moins des surfaces de l'une et de l'autre des lentilles est une surface de révolution asphérique.

Préférentiellement, il s'agit d'une surface de révolution ayant pour génératrice une conique.

Il s'avère, en effet, que, par un choix approprié, pour l'une et l'autre des surfaces concernées, de la constante conique d'une telle surface, il est avantageusement possible de réaliser un système optique permettant l'obtention, sur la rétine de l'oeil, d'images corrigées de la coma et de l'aberration sphérique longitudinale, l'aberration sphérique longitudinale de l'ensemble que forme avec l'oeil ce système optique pouvant par exemple être inférieure à celle d'une oeil emmétrope dans les mêmes conditions de vision.

Plus précisément, il est avantageusement possible de réaliser, suivant l'invention, un système optique qui, pour un champ objet d'une étendue déterminée, de l'ordre par exemple de 2 x 10 mm de diamètre, est avantageusement sensiblement aplanétique, l'image d'un objet situé dans un plan se formant exactement sur la rétine, elle-même assimilée à un plan, sans coma ni aberration sphérique longitudinale, avec, pour des champs supérieurs, la seule subsistance d'un peu de coma et d'un peu d'astigmatisme.

Ainsi, pour un champ inférieur à 2 x 10 mm, ce système optique est apte à donner d'un point une tache de diamètre de l'ordre de 5 microns, ce qui correspond au diamètre des détecteurs rétiniens, et est donc garant d'une bonne qualité pour les images obtenues.

En outre, comme le champ image qui en résulte est en pratique supérieur à la fovéa, alors même que c'est sur le centre de cette fovéa que se fait l'image du point de fixation lorsque l'on fixe du regard un objet, il est avantageusement possible, avec le système optique suivant l'invention, d'avoir une bonne qualité d'image pour toute la zone de vision, ou zone centrale, qui correspond à cette fovéa et dont dépend la précision de la perception obtenue.

En bref, il est avantageusement possible, avec le système optique suivant l'invention, d'obtenir, sur la rétine de l'oeil concerné, des images "propres", c'est-à-dire pratiquement sans aberration.

Les caractéristiques et avantages de l'invention ressortiront d'ailleurs de la description qui va suivre, à titre d'exemple, en référence aux dessins schématiques annexés sur lesquels :

la figure 1 est une vue en élévation-coupe d'un système optique suivant l'invention ;

la figure 2 est, à échelle supérieure, une vue en plan de la lentille intra-oculaire que comporte ce système optique ;

la figure 3 est une vue en coupe axiale de cette lentille intra-oculaire, suivant la ligne III-III de la figure 2 ;

la figure 4 est formée par un ensemble de diagrammes illustrant, à une première échelle, les résultats obtenus, dans le plan tangentiel, avec le système optique suivant l'invention ;

la figure 5 est formée par un ensemble de diagrammes illustrant, à la même échelle que celle de la figure 4, les résultats obtenus, dans le plan sagittal, par ce système optique ;

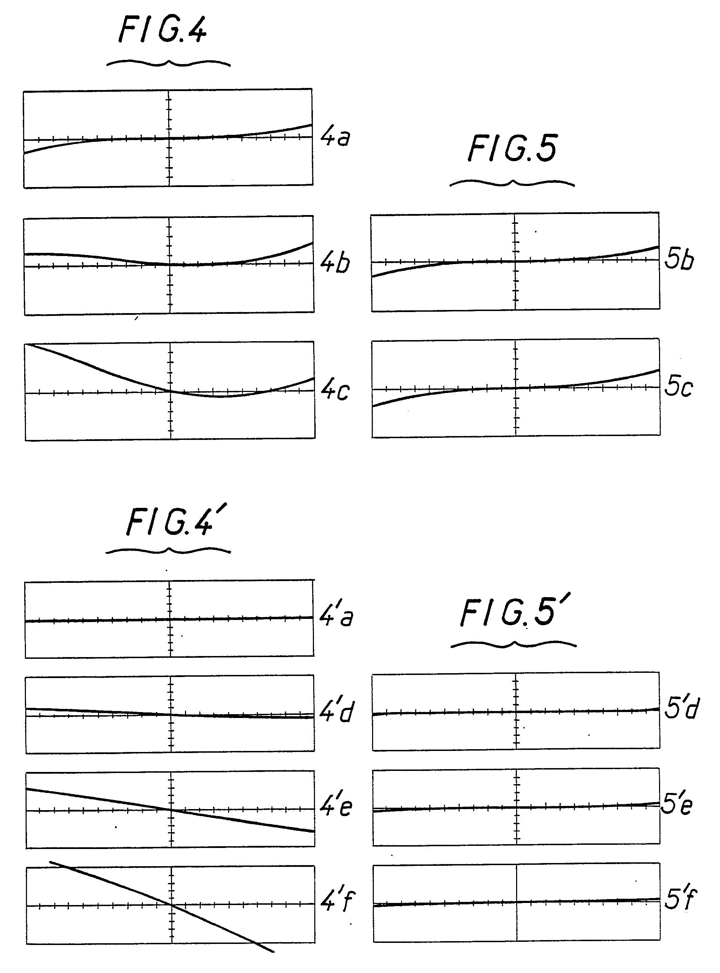

la figure 4′ est, à une échelle différente de celle de la figure 4, un ensemble de diagrammes dont le premier correspond à celui de même rang de cette figure 4 et dont les autres viennent en complément de ceux de celle-ci ;

la figure 5′ est un ensemble de diagrammes qui, à l'échelle de ceux de la figure 4′, viennent en complément de ceux de la figure 5.

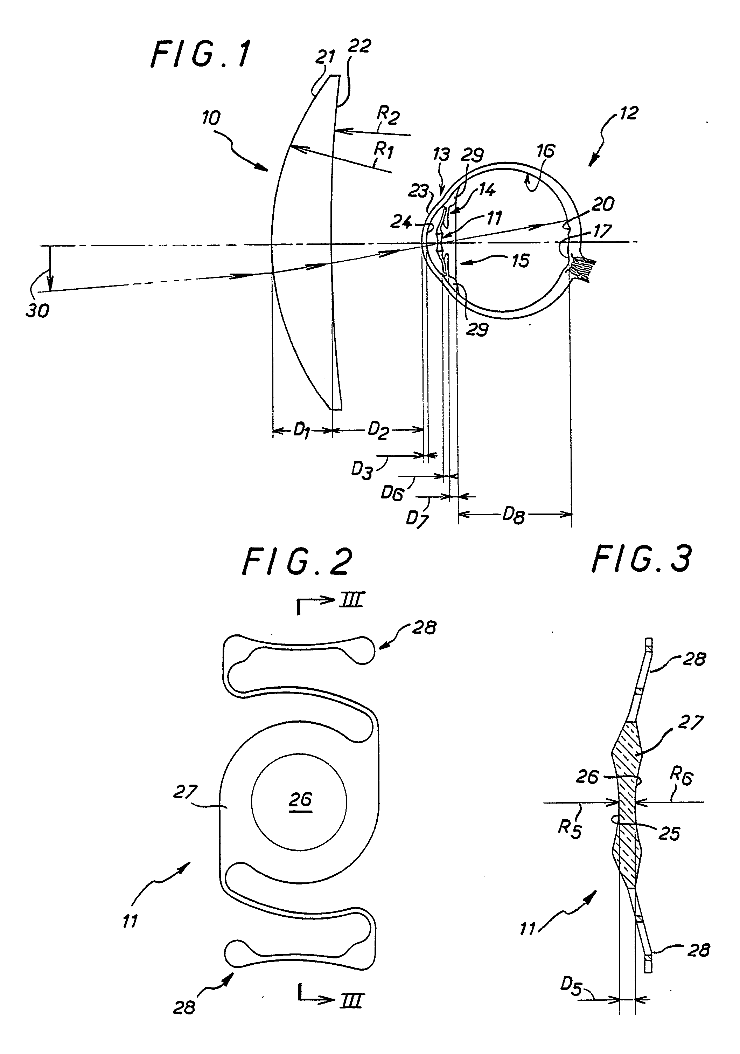

Tel qu'illustré sur la figure 1, le système optique suivant l'invention, qui est destiné à l'amélioration de la vision d'une personne atteinte de dégénérescence maculaire, comporte, en combinaison, de manière connue en soi, deux lentilles 10, 11, à savoir d'une part, une lentille ophtalmique 10, de forte puissance positive, disposée devant l'oeil 12 à traiter, et, d'autre part, une lentille intra-oculaire 11, de forte puissance négative, implantée dans cet oeil 12, après ablation de son cristallin.

S'agissant de l'oeil 12, on reconnaît, sur la figure 1, en 13 la cornée, en 14 l'iris, en 15 le sac ayant initialement contenu le cristallin, et en 16 la rétine, avec, au-dessus de la papille 17 disposée au droit du nerf optique 18, la fovéa 20.

La face avant 21 de la lentille ophtalmique 10 est convexe.

A supposer, pour l'instant, sphérique cette face avant 21, soit $R_1$ son rayon.

Dans la forme de réalisation représentée, la face arrière 22 de la lentille ophtalmique 10 est sensiblement plane, tout en étant légèrement concave.

Il s'agit d'une surface sphérique.

Soit $R_2$ son rayon.

Soit $D_1$ la distance séparant l'un de l'autre, sur l'axe de l'ensemble, les dioptres correspondants.

Soit $n_1$, enfin, l'indice de réfraction du matériau constitutif de cette lentille ophtalmique 10.

On supposera ci-après que cette lentille ophtalmique 10 est implantée à une distance $D_2$ de la cornée 13.

Soit $R_3$ le rayon de la face antérieure 23 de cette cornée 13, $R_4$ le rayon de sa face postérieure 24, $D_3$ la distance séparant l'une de l'autre, sur l'axe de l'ensemble, ces deux faces 23, 24, et $n_3$ l'indice de réfraction entre celles-ci.

Dans la forme de mise en oeuvre représentée, qui est une forme préférentielle de mise en oeuvre, la lentille intra-oculaire 11 est implantée dans la chambre antérieure de l'oeil 12, c'est-à-dire dans la partie de celui-ci qui s'étend entre sa cornée 13 et son iris 14.

Soit $D_4$ la distance séparant axialement de la cornée 13 cette lentille intra-oculaire 11, et $n_4$ l'indice de réfraction de l'humeur acqueuse correspondante.

En pratique, la lentille intra-oculaire 11 est une lentille bi-concave, figures 2, 3.

Comme la lentille ophtalmique 10, il peut s'agir d'une lentille en polyméthacrylate de méthyle.

Soit $R_5$ le rayon de sa face avant 25, à supposer, pour l'instant, sphérique cette face avant 25, $R_6$ le rayon de sa face arrière 26, $D_5$ la distance séparant l'une de l'autre, sur l'axe de l'ensemble, ces deux faces 25, 26, et $n_5$ l'indice de réfraction de son matériau constitutif.

De manière connue en soi, cette lentille intra-oculaire 11 comporte, annulairement, autour de ses faces 25, 26, un épaississement 27, et, issus de celui-ci, en positions diamétralement opposées l'un par rapport à l'autre, deux bras élastiquement déformables 28, qui, chacun d'allure en S, sont propres à son appui sur les corps ciliaires 29 de l'oeil 12, à la racine de l'iris 14 de celui-ci.

La partie utile de cette lentille intra-oculaire 11, formée par ses seules faces 25, 26, a un diamètre relativement réduit, de l'ordre par exemple de 3 mm.

Soit $D_6$ la distance séparant axialement cette lentille intra-oculaire 11 de l'iris 14, $D_7$ la distance séparant axialement le sac 15 de cet iris 14, et $D_8$ la distance séparant axialement la rétine 17 de ce sac 15.

Soit, enfin, $n_5$ l'indice de réfraction de l'humeur vitrée présente entre le sac 15 et la rétine 17, et $R_7$ le rayon de cette dernière.

Suivant l'invention, l'une au moins des surfaces de l'une et de l'autre des lentilles 10, 11 est une surface de révolution asphérique.

De préférence, cette surface de révolution asphérique a pour génératrice une conique, et il en est ainsi pour l'une et l'autre des deux lentilles 10, 11 concernées.

De préférence, également, celle des faces d'une telle lentille qui est une surface de révolution asphérique en est la face avant.

Il s'agit donc de la face 21 pour la lentille ophtalmique 10, et de la face 25 pour la lentille intra-oculaire 11.

Ainsi qu'on le sait, l'équation d'une surface de révolution asphérique ayant pour génératrice une conique peut s'écrire de la manière suivante :

$$Z = \frac{\dfrac{r^2}{R}}{1 + \sqrt{1 - (1 + K)\dfrac{r^2}{R^2}}}$$

expression dans laquelle :
Z est la coordonnée suivant l'axe de l'ensemble,
$r^2 = X^2 + Y^2$, X et Y étant les coordonnées dans un plan perpendiculaire à cet axe,
R est le rayon osculateur de la surface au centre,
et K est la constante conique permettant de déduire de la surface sphérique de base de rayon R la surface à génératrice formée par une conique recherchée.

Suivant l'invention l'équation de la face avant 21 de la lentille ophtalmique 10 est donc la suivante :

$$Z = \frac{\dfrac{r^2}{R_1}}{1 + \sqrt{1 - (1 + K_1)\dfrac{r^2}{R_1{}^2}}}$$

et l'équation de la face avant 25 de la lentille intra-oculaire 11 est la suivante :

$$Z = \frac{\dfrac{r^2}{R_5}}{1 + \sqrt{1 - (1 + K_5)\dfrac{r^2}{R_5{}^2}}}$$

De bons résultats sont obtenus, quant à la qualité des images formées sur la rétine 16, lorsque, par un choix approprié de la constante conique, il est fait en sorte que, tant pour la lentille ophtalmique 10 que pour la lentille intra-oculaire 11, la génératrice de la surface asphérique est une ellipse.

Pour un système optique dont la lentille intra-oculaire 11 a une puissance globale de - 50 dioptries, et pour la vision de près, c'est-à-dire pour une vision portant sur un objet 30 supposé être à environ 33,3 cm de l'oeil 12 concerné, de bons résultats sont obtenus lorsque la constante optique $K_1$ de la lentille ophtalmique 10 est comprise entre - 0,1 et - 0,3 pour un indice de réfraction $n_1$ égal à 1,523, et lorsque, conjointement, la constante conique $K_5$ de la lentille intra-oculaire 11 est comprise entre - 2 et - 4 pour un indice de réfraction $n_5$ égal à 1,492.

Mais des résultats particulièrement satisfaisants sont obtenus lorsque, pour la lentille ophtalmique 10, la constante conique $K_1$ est égale à - 0,185790, et que, conjointement, pour la lentille intra-oculaire 11, la constante conique $K_5$ est égale à - 2,699500.

Bien entendu, les valeurs des constantes coniques $K_1$ et $K_5$ sont en relation directe avec les valeurs des indices de réfraction $n_1$, $n_5$ des lentilles 10 et 11 concernées.

Ainsi, à un autre compte de valeur des indices de réfraction ($n_1$, $n_5$) sera associé un autre couple de valeurs des constantes coniques ($K_1$, $K_5$)

On résumera, dans le tableau T suivant, les valeurs numériques d'un système optique suivant l'invention, dont la lentille intra-oculaire 11 a une puissance globale égale à - 50 dioptries, et pour une distance objet de 33,3 cm, en indiquant, que pour les calculs correspondants, il a été supposé

EP 0 362 004 A1

que la face antérieure 23 de la cornée 13 constituait elle aussi une surface asphérique de révolution ayant pour génératrice une ellipse, avec une constante conique $K_2$ égale à - 0,26000.

T

| | RAYONS mm | DISTANCES AXIALES mm | INDICES DE REFRACTION | CONSTANTE CONIQUE |
|---|---|---|---|---|
| lentille ophtalmique 10 | $R_1$ = 18,155 <br> $R_2$ = 400,000 | $D_1$ = 10 | $n_1$ = 1,52300 | $K_1$ = -0,185790 |
| air | | $D_2$ = 13 | | |
| cornée 13 | $R_3$ = 7,720 <br> $R_4$ = 6,500 | $D_3$ = 0,550 | $n_3$ = 1,36700 | $K_3$ = -0,26000 |
| humeur acqueuse | | $D_4$ = 2,000 | $n_4$ = 1,33740 | |
| lentille intra-oculaire 11 | $R_5$ = -6,200 <br> $R_6$ = 6,200 | $D_5$ = 0,500 | $n_5$ = 1,49200 | $K_5$ = -2,699500 |
| humeur acqueuse | | $D_6$ = 0,550 | $n_4$ = 1,33740 | |
| iris 14 | $\infty$ | | | |
| humeur acqueuse | | $D_7$ = 2,000 | $n_4$ = 1,33740 | |
| sac 15 | $\infty$ | | | |
| humeur vitrée | | $D_8$ = 18,341 | $n_6$ = 1,33600 | |
| rétine 17 | $R_7$ = -12,000 | | | |

Les figures 4, 4', 5 et 5' illustrent les résultats particulièrement satisfaisants obtenus avec un tel système optique.

Elles représentent l'aberration transverse donnée, dans le plan image, par faisceau lumineux issu d'un point objet situé à 33,3 cm.

Pour les figures 4 et 4', la nappe de rayons lumineux est dans le plan tangentiel, en pratique le plan de figure.

Pour les figures 5 et 5', elle est dans le plan sagittal, perpendiculaire au précédent.

Sur les diagrammes formant ces figures, l'échelle utilisée pour les abscisses est toujours la même, le rayon de la pupille d'entrée du système étant supposé y correspondre à l'unité.

Sur les figures 4 et 5, l'échelle des ordonnées est supposée correspondre à une valeur maximale égale à 0,005 mm.

Par contre, sur les figures 4' et 5', elle est supposée correspondre à une valeur maximale égale à 0,1 mm.

Sur les diagrammes 4a et 4'a le point objet est sur l'axe de l'ensemble.

Sur les autres diagrammes, il est au contraire écarté de cet axe, de 5 mm pour les diagrammes 4b et 5b, de 10 mm pour les diagrammes 4c et 5c, de 20 mm pour les diagrammes 4'd et 5'd, de 40 mm pour les diagrammes 4'e et 5'e, et de 60 mm pour les diagrammes 4'f et 5'f.

Ainsi qu'il apparaîtra à l'homme de l'art, il résulte de l'observation de ces diagrammes que le système optique répondant aux valeurs numériques données dans le tableau T précédent est sensiblement aplanétique pour un champ objet de 2 x 10 mm de diamètre, l'aberration sphérique longitudinale de l'ensemble qu'il forme avec l'oeil 12 concerné, aussi bien que la coma, étant sensiblement nulles pour un tel champ.

En pratique, pour ce champ de 2 x 10 mm de diamètre, l'image d'un point est une tache de diamètre égale à 5 microns, ce qui correspond au diamètre des détecteurs rétiniens.

Les diagrammes des figures 4, 4', 5 et 5' montrent également que, pour un champ objet allant de 2 x 20 mm à 2 x 60 mm, les courbes d'aberration sont presque planes.

Il n'y a plus d'abberation longitudinale sphérique, et il ne reste qu'un peu de coma, surtout pour un champ objet de 2 x 60 mm de diamètre.

En effet, l'aberration donnée par un tel système optique relève pour l'essentiel de l'astigmatisme, et il est possible de s'en satisfaire.

Dans tous les cas, l'aberration sphérique longitudinale reste largement inférieure à celle d'un oeil emmétrope dans les mêmes conditions de vision.

Bien entendu, la présente invention ne se limite pas à la forme de réalisation décrite et représentée, mais englobe toute variante d'exécution.

En particulier, l'une et l'autre des surfaces de l'une au moins des lentilles mises en oeuvre peuvent être asphériques.

## Revendications

1. Système optique pour l'amélioration de la vision d'une personne atteinte de dégénérescence maculaire, du genre comportant, en combinaison, deux lentilles (10, 11), à savoir, d'une part, une lentille ophtalmique (10) de forte puissance positive disposée devant l'oeil à traiter, et, d'autre part, une lentille intra-oculaire (11) de forte puissance négative implantée dans cet oeil après ablation de son cristallin, caractérisé en ce que l'une au moins des surfaces de l'une et de l'autre des lentilles (10, 11), est une surface de révolution asphérique.

2. Système optique suivant la revendication 1, caractérisé en ce que, pour l'une au moins des lentilles (10, 11), la surface de révolution asphérique a pour génératrice une conique.

3. Système optique suivant la revendication 2, caractérisé en ce que, pour l'une et l'autre des lentilles (10, 11), la surface de révolution asphérique a pour génératrice une conique.

4. Système optique suivant la revendication 3, caractérisé en ce que, pour l'une et l'autre des lentilles (10, 11), la surface de révolution asphérique a pour génératrice une ellipse.

5. Système optique suivant la revendication 4, caractérisé en ce que, pour une puissance globale de - 50 dioptries pour la lentille intra-oculaire (11), la constante conique est comprise entre - 0,1 et - 0,3 pour une lentille ophtalmique (10) d'indice de réfraction ($n_1$) égal à 1,523, et elle est comprise entre - 2 et - 4 pour une lentille intra-oculaire (11) d'indice de réfraction ($n_5$) égal à 1,492.

6. Système optique suivant la revendication 5, caractérisé en ce que, la constante conique est égale à - 0,185790 pour la lentille ophtalmique (10), et, elle est égale à - 2,699500 pour la lentille intra-oculaire (11).

7. Système optique suivant l'une quelconque des revendications 1 à 6, caractérisé en ce que celle des faces d'une des lentilles (10, 11) qui est une surface de révolution asphérique en est la face avant.

8. Système optique suivant l'une quelconque des revendications 1 à 7, caractérisé en ce que la lentille intra-oculaire (11) est implantée dans la chambre antérieure de l'oeil concerné.

9. Système optique suivant l'une quelconque des revendications 1 à 8, caractérisé en ce que l'aberration sphérique longitudinale de l'ensemble qu'il forme avec l'oeil concerné est inférieure à celle d'un oeil emmétrope dans les mêmes conditions de vision.

10. Système optique suivant l'une quelconque des revendications 1 à 9, caractérisé en ce que, pour un champ objet de 2 x 10 mm de diamètre, il est sensiblement aplanétique.

FIG.1

FIG.2        FIG.3

EP 0 362 004 A1

FIG.4

4a

4b

4c

FIG.5

5b

5c

FIG.4′

4′a

4′d

4′e

4′f

FIG.5′

5′d

5′e

5′f

| | Office européen des brevets | **RAPPORT DE RECHERCHE EUROPEENNE** | Numero de la demande |
|---|---|---|---|
| | | | EP 89 40 2402 |

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| Y | WO-A-8 301 566 (KOESTER) <br> * Page 7, ligne 15 - page 13, ligne 18; figures 1-3,7 * | 1,2,7 | G 02 C 7/02 <br> A 61 F 2/16 |
| A | | 5 | |
| | --- | | |
| Y | US-A-4 504 982 (BURK) <br> * En entier * | 1,2,7 | |
| A | | 3-6 | |
| | --- | | |
| A | FR-A-2 509 482 (O.W.G.R.) <br> * Page 6, ligne 36 - page 9, ligne 37; figure 1 * | 1-3,5-7 | |
| | --- | | |
| A | US-A-4 666 446 (KOZIOL) <br> * Colonne 1, lignes 25-51; colonne 4, ligne 45 - colonne 5, ligne 1; figures 3,6,13,15 * | 1,5,10 | |
| | --- | | |
| A | FR-A-1 089 254 (S.I.C.O.L.) <br> * En entier * | 1-5,7 | DOMAINES TECHNIQUES RECHERCHES (Int. Cl.5) |
| | --- | | |
| A | US-A-4 710 193 (VOLK) <br> * Colonne 3, ligne 56-68; colonne 5, lignes 61-67; colonne 14, lignes 12-19; colonne 19, ligne 15 - colonne 20, ligne 9; figures 2,7,8 * | 1-5,7, 10 | A 61 F <br> G 02 C |
| | --- | | |
| A | EP-A-0 162 573 (HECHT) <br> * Abrégé; figures 1-6,11,17A * | 8 | |
| | --- | | |
| A | EP-A-0 242 043 (ALLERGAN) <br> * Colonne 2, lignes 28-30; figure 1a * | 8 | |
| | --- | | |
| A | EP-A-0 157 476 (PILKINGTON) <br> * Abrégé * | 9 | |
| | ---       -/- | | |

Le présent rapport a été établi pour toutes les revendications

| Lieu de la recherche | Date d'achèvement de la recherche | Examinateur |
|---|---|---|
| LA HAYE | 28-11-1989 | KLEIN C. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

............................................................................................
& : membre de la même famille, document correspondant

EPO FORM 1503 03.82 (P0402)

Office européen
des brevets

# RAPPORT DE RECHERCHE EUROPEENNE

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | GB-A- 20 620 (CARL ZEISS)(A.D.1910) --- | | |
| A | WO-A-8 303 481 (LEVY) --- | | |
| A | EP-A-0 195 881 (PHARMACIA) ----- | | |

DOMAINES TECHNIQUES RECHERCHES (Int. Cl.5)

Le présent rapport a été établi pour toutes les revendications

| Lieu de la recherche | Date d'achèvement de la recherche | Examinateur |
|---|---|---|
| LA HAYE | 28-11-1989 | KLEIN C. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
 
& : membre de la même famille, document correspondant

EPO FORM 1503 03.82 (P0402)